# EUROPEAN PATENT SPECIFICATION

(11) **EP 1 244 775 B1**
(45) Date of publication and mention of the grant of the patent: **08.10.2008**
(21) Application number: 00985517.2
(22) Date of filing: 08.12.2000
(51) Int. Cl.: C12N 9/02, C12N 9/10, C12N 15/62, C12N 15/54, C12N 15/63, C12N 5/10, G01N 33/547

(54) **MOLECULAR DISPLAY ON MULTIMERIC PROTEIN SCAFFOLDS DERIVED FROM THE E2 COMPONENT OF THE ALPHA-KETOACID DEHYDROGENASE**
MOLEKULARDISPLAY AUF MULTIMEREN PROTEINGERÜSTEN, DIE VON DER E2 KOMPONENTE DER ALPHA-KETOSÄURE-DEHYDROGENASE STAMMEN
AFFICHAGE MOLECULAIRE SUR DES ECHAFAUDAGES PROTEINIQUES MULTIMERES

(30) Priority: 09.12.1999 GB 9929151
(43) Date of publication of application: 02.10.2002
(73) Proprietor: Accentus Plc, London WC2N 5HR (GB)
(72) Inventor: PERHAM, Richard, Nelson, Cambridge, Cambridgeshire CB3 9LL (GB); DOMINGO, Gonzalo, Jose, E-46111 Rocafort (ES)
(74) Representative: Mansfield, Peter Turquand
(86) International application number: PCT/GB2000/004720
(87) International publication number: WO 2001/042439

(56) References cited:
- WO-A-97/49720
- ALLEN MARK D ET AL: "The catalytic domain of dihydrolipoyl acetyltransferase from the pyruvate dehydrogenase multienzyme complex of Bacillus stearothermophilus." FEBS LETTERS, vol. 413, no. 2, 1997, pages 339-343, XP000997933 ISSN: 0014-5793 cited in the application
- WYNN R MAX ET AL: "In vitro reconstitution of the 24-meric E2 inner core of bovine mitochondrial branched-chain alpha-keto acid dehydrogenase complex: Requirement for chaperonins GroEL and GroES." BIOCHEMISTRY, vol. 33, no. 30, 1994, pages 8962-8968, XP000993026 ISSN: 0006-2960 cited in the application
- HANEMAAIJER R ET AL: "THE GENE ENCODING DIHYDROLIPOYL TRANSACETYLASE FROM AZOTOBACTER-VINELANDII EXPRESSION IN ESCHERICHIA-COLI AND ACTIVATION AND ISOLATION OF THE PROTEIN" EUROPEAN JOURNAL OF BIOCHEMISTRY, vol. 181, no. 1, 1989, pages 47-54, XP000997906 ISSN: 0014-2956
- LAWSON J E ET AL: "FUNCTIONAL ANALYSIS OF THE DOMAINS OF DIHYDROLIPOAMIDE ACETYLTRANSFERASE FROM SACCHAROMYCES-CEREVISIAE" BIOCHEMISTRY, vol. 30, no. 47, 1991, pages 11249-11254, XP000993025 ISSN: 0006-2960
- LIU SHENGJIANG ET AL: "Recombinant expression and evaluation of the lipoyl domains of the dihydrolipoyl acetyltransferase component of the human pyruvate dehydrogenase complex." ARCHIVES OF BIOCHEMISTRY AND BIOPHYSICS, vol. 316, no. 2, 1995, pages 926-940, XP000997905 ISSN: 0003-9861
- P MALIK AND R N PERHAM: "Simultaneous display of different peptides on the surface of filamentous bacteriophages" NUCLEIC ACIDS RESEARCH,GB,OXFORD UNIVERSITY PRESS, SURREY, vol. 25, no. 4, 1997, pages 915-916, XP002132956 ISSN: 0305-1048 cited in the application
- DOI N ET AL: "SCREENING OF CONFORMATIONALLY CONSTRAINED RANDOM POLYPEPTIDE LIBRARIES DISPLAYED ON A PROTEIN SCAFFOLD" CMLS CELLULAR AND MOLECULAR LIFE SCIENCES,CH,BIRKHAUSER VERLAG, BASEL, vol. 54, no. 5, May 1998 (1998-05), pages 394-404, XP000891835 ISSN: 1420-682X
- YAFFE, MICHAEL B. ET AL: "Mapping specificity determinants for protein-protein association using protein fusions and random peptide libraries" METHODS ENZYMOL. (APPLICATIONS OF CHIMERIC GENES AND HYBRID PROTEINS, PT. C), vol. 328, 2000, pages 157-170, XP000997949
- DOMINGO GONZALO J ET AL: "Multiple display of peptides and proteins on a macromolecular scaffold derived from a multienzyme complex." JOURNAL OF MOLECULAR BIOLOGY, vol. 305, no. 2, 12 January 2001 (2001-01-12), pages 259-267, XP000998078 ISSN: 0022-2836

## Description

The present invention relates to display of peptides and polypeptides. More particularly, the invention relates to display on multienzyme complex proteins, especially a 2-oxo acid dehydrogenase multienzyme complex. The display of peptides and polypeptides is useful in a variety of contexts, including screening for those which bind target proteins of interest or are bound by target antibodies of interest, or which have other desirable properties, and in the elicitation of immune responses, e.g. for vaccination. In embodiments of the present invention, advantages arise from the ability to display a variety of different numbers of peptides or polypeptides on a single complex, and the ability to display a variety of different peptides or polypeptides on the same complex.

The development of molecular biology techniques has encouraged manipulation of large complex biological systems for the display of exogenous biologically active molecules on their surfaces. Most commonly, viruses have been engineered for the presentation of epitopes, useful in epitope mapping, in generating antibodies, in vaccination contexts, and for the presentation of whole proteins or domains, such as antibodies, enzymes or hormone receptors. See for example Greenwood et al., (1991) Journal of Molecular Biology 220: 821-827; Sutter and Moss (1992) PNAS (USA) 89: 10847-10851; Willis et al., (1993) Gene 128: 79-83; Cortese et al., (1994) Trends in Biotechnology 12: 262-267; Veronese et al., (1994) Journal of Molecular Biology 243: 167-172; Meola et al., (1995) Journal of Immunology 154: 3162-3172; Kay et al., (1996) Phage Display of Peptides and Proteins: A Laboratory Manual, Academic Press, and Bendahmane et al., (1999) Journal of Molecular Biology 290: 9-20, also WO92/07077. Display of polypeptide chains on viral surfaces has the advantage that the genetic information encoding the displayed polypeptide of interest is intrinsically coupled to that same polypeptide. This has permitted the creation of libraries of displayed peptides or polypeptides from which molecules of interest can be selected and identified. Polypeptide domains have been displayed for selection purposes, e.g. of antibodies and other proteins (Kay et al., (1996) Phage Display of Peptides and Proteins: A Laboratory Manual, Academic Press), and enzymes (see e.g. Atwell and Wells (1999) PNAS (USA) 96: 9497-9502). However, these systems are often limited by the number of different exogenous molecules that can be displayed on any single virion (Malik and Perham (1997) Nucleic Acids Research 25: 915-916), and the number of copies of the same exogenous polypeptide displayed on the surface of the virion is often compromised by the size of the polypeptide and the need to be compatible with the assembly of the scaffold (Malik et al., (1996) Journal of Molecular Biology 260: 9-21).

The present invention is based on creation of a display system for peptides and polypeptides, useful in the contexts in which existing display systems have been employed, but also in others. A number of advantages may be achieved using embodiments of the present invention, as disclosed herein.

The invention employs one or more components of a multienzyme complex especially a 2-oxo acid dehydrogenase multienzyme complex, for example a pyruvate dehydrogenase (PDH) complex such as of *Bacillus stearothermophilus.*

Members of the 2-oxo acid (or α-keto acid) dehydrogenase family of multienzyme complexes, which are responsible for the oxidative decarboxylation of 2-oxo acids, are generally composed of three enzymes: E1, a 2-oxo acid decarboxylase; E2, an acyl transferase; and E3, a dihydrolipoyl dehydrogenase. The E1 component catalyses the oxidative decarboxylation of 2-oxo acids and the subsequent reductive acylation of a lipoyl group bound to E2. The acyl group is then transferred to CoA in a reaction catalysed by the E2 component, and the reduced lipoyl group is reoxidised by the E3 component with concomitant reduction of NAD. This family of multienzyme complexes includes the pyruvate dehydrogenase (PDH) complex, the 2-oxoglutarate (α-ketoglutarate) dehydrogenase (OGDH) complex, and the branched-chain 2-oxo acid (α-keto acid) dehydrogenase (BCODH) complex. The acetoin dehydrogenase (ADH) - complex is similar. For reviews see Perham (1991) Biochemistry 30, 8501-8512 and de Kok et al., (1998) Biochimica et Biophysica Acta 1385: 353-366. Such enzymes are found in Gram-negative and Gram-positive bacteria, and in eukaryotes including yeast.

The E2 component forms the structural core to which the E1 and E3 sub-units bind tightly but non-covalently in peripheral positions. Some complexes (notably from eukaryotes) also have a fourth polypeptide chain, involved in binding E3, called the E3-binding protein (E3-BP) or protein X, which is associated with the E2 core. The fully assembled enzyme complex is typically between 5 and 10 MDa in molecular mass. The E2 polypeptide chains form trimers, which in turn assemble to form either a cube-like core composed of 24 E2 chains arranged with octahedral symmetry, or a pentagonal dodecahedral core composed of 60 E2 chains with icosahedral symmetry. The symmetry is dependent on the source and the nature of the complex.

The pyruvate dehydrogenase (PDH) complex of *Bacillus stearothermophilus* is a member of this family of multienzyme complexes and has been fully reconstituted from recombinant proteins *in vitro* (Lessard et al., (1998) European Journal of Biochemistry, 258: 491-501). The heterotetrameric (α₂β₂) E1p component (150 kDa) and the dimeric E3 component (90 kDa) assemble around the pentagonal dodecahedral E2p core, which consists of 60 E2 chains. See e.g. Berg and de Kok (1997) Biological Chemistry 378: 617-634, Patel and Roche (1990) FASEB Journal 4: 3224-3233 and Perham (1991) Biochemistry 30: 8501-8512.

The E2p polypeptide chain of *B*. *stearothermophilus* is composed of three independently folded domains separated by flexible linker regions: a lipoyl domain of 9.5 kDa, a peripheral subunit-binding domain of 5.3 kDa, and a catalytic core domain of 28 kDa. The catalytic core domain is responsible for the formation of the pentagonal dodecahedral protein scaffold. The X-ray crystal structure of this E2p domain has been elucidated (Izard et al., (1999) Proceedings of the National Academy of Sciences USA, 96: 1240-1245). The structure has an outer diameter of 240 Å, the twelve five-fold faces of the dodecahedron have openings of 50 Å in diameter into the central cavity, which in turn has a diameter of 180 Å. These dimensions render the E2 core easily visible by electron microscopy.

The *B*. *stearothermophilus* E2 protein component can be expressed and purified from *E*. *coli* in yields of over 15 mgs/l of cell culture. The E2 core is stable in aqueous media at room temperature, and can be refolded from 6M guanidine hydrochloride to yield 95% active enzyme (Allen & Perham (1997) FEBS Letters 413: 339-343; Lessard et al., (1998) European Journal of Biochemistry, 258: 491-501).

All 2-oxo acid dehydrogenase complexes follow this pattern described for the *B*. *stearothermophilus* PDH complex, though variations in the number of lipoyl domains per E2 chain and structure of: the E1 component are possible (Perham (1991) Biochemistry 30, 8501-8512 and de Kok et al., (1998) Biochimica et Biophysica Acta 1385: 353-366).

In the present invention, a truncated form of an E2 core protein of a 2-oxo acid dehydrogenase multienzyme complex is provided as a scaffold for display of a peptide or polypeptide, either by means of fusion (through covalent linkage) of the peptide or polypeptide to the truncated E2 protein, or by means of non-covalent interaction. The truncated E2 core protein comprises at least a portion able to assemble into a core structure (which may be octahedral or icosahedral as discussed). Such a portion is generally (i.e. the truncated E2 core protein generally comprises) the catalytic core (third) domain.

An aspect of the invention provides a population or library of assembled cores of a 2-oxo acid dehydrogenase multienzyme complex as set out in claim 1.

A truncated E2 core protein of a 2-oxo acid dehydrogenase multienzyme complex may assemble into a core structure of the complex. Such a truncated E2 core protein generally lacks the lipoyl domain of full-length E2 core protein and may lack the peripheral subunit-binding domain.

Where the peptide or polypeptide to be displayed is provided as a fusion with the truncated E2 protein, the site of truncation may be such that the E2 protein lacks the lipoyl domain and the peripheral subunit-binding domain. Preferred such sites of truncation include all residues in the linker region between the peripheral subunit-binding domain and the catalytic core domain. With reference to the E2 core protein of the pyruvate dehydrogenase (PDH) complex of *B*. *stearothermophilus,* preferred such truncated E2 proteins (or E2 portions) for use in the present invention consist of residues Ala167 to Ala427 or Lys187 to Ala427, according to *B*. *stearothermophilus* E2p sequence deposited in the protein database (ODP2_BACST, Accession number P11961). Where the peripheral subunit-binding domain is included, preferred sites of truncation include all residues from the N-terminus of E2 to the residue at the N-terminal end of the peripheral subunit-binding domain. With reference to the E2 core protein of pyruvate dehydrogenase (PDH) complex of *B*. *stearothermophilus,* preferred such truncated E2 proteins (or E2 portions) for use in the present invention consist of residues Lys86 to Ala427 or Arg127 to Ala427, according to the B. *stearothermophilus* E2p sequence deposited in the protein database (ODP2_BACST, Accession number P11961).

A fusion may comprise (i) a portion of a 2-oxo acid dehydrogenase multienzyme complex E2 protein component which assembles into a core structure of the complex and (ii) a heterologous peptide or polypeptide.

A linker molecule may be introduced between the truncated E2 core protein and the displayed molecule. This linker can be in the form of the naturally existing polypeptide linker between the E2 core domain and the peripheral subunit-binding domain or in the form of an unrelated linker polypeptide chain.

Chemical modification of the E2 protein can be used to attach one or more molecules other than peptides or polypeptides. These molecules can be attached to the portion of the E2 or other multi enzyme complex component used as a scaffold or to a peptide or polypeptide displayed on the scaffold.

The insertion site for the heterologous peptide or polypeptide is generally at the N-terminus of the E2 portion employed for display. An advantage of fusion at the N-terminus is the ease of N-terminal sequencing, e.g. to determine the amino acid sequence of a peptide insert found by screening to have a desired property. This offers an alternative to screening clones and retrieving the encoding nucleic acid.

Other appropriate insertion sites include at the C-terminus of the E2 core domain (the C-terminus of the normal intact E2) as exists in some naturally occurring PDH complexes (e.g. Powles and Rawlings (1997) Microbiology 143, 2189-2195; Usuda et al., (1996) Microbiology 142, 3347-3354), or at the N-terminus of intact or truncated E3-binding proteins (E3-BPs) of the eukaryotic multienzyme complexes. In addition, foreign molecules may be attached to the E2 core surface at any desired position, for example by chemical reaction with a cysteine residue, which may be introduced appropriately by site-directed mutagenesis into the E2 core domain or peripheral subunit-binding domain or lipoyl domain.

Generally a truncated E2 core protein or a fusion protein as disclosed is provided by expression from encoding nucleic acid. Recombinant DNA technology may be employed to generate such a coding sequence using standard techniques (discussed further below).

Nucleic acid encoding such a truncated E2 protein or a fusion as disclosed is provided as a further aspect of the present invention, also vectors, recombinant vectors such as expression vectors, and host cells transformed with such nucleic acid or vector.

The truncated E2 protein component of an assembled core of a 2-oxo acid dehydrogenase multienzyme complex as disclosed may assemble to provide an octahedral or icosahedral core. The core may comprise one or more E2 protein components comprising a portion which assembles to form the core wherein the portion is fused to a heterologous peptide or polypeptide for display of the peptide or polypeptide. The peptide or polypeptide is displayed on the complex by virtue of its fusion (generally at its C-terminus) with said component.

The number of fusions within a complex may be varied. In an icosahedral core comprising 60 copies of the component, 1-60 of the components may be provided as fusions. In an octahedral core comprising 24 copies of the component, 1-24 of the components may be preferred as fusions. Preferred numbers of fusions include 1, 2, 3, 4, 5 or more. A small copy number is good for selection purposes, since multiple interactions with the targeting ligand (e.g. an immobilised antibody) are eliminated or greatly diminished.. A high copy number (up to the full 24 or 60) is important in, for example, antigen presentation for vaccine purposes.

A core may be provided displaying more than one different peptide or polypeptide. Such complexes are readily provided by assembly of mixtures of components fused to different peptides or polypeptides. This allows for provision of complexes displaying a plurality of epitopes which may be useful for raising an immune response, e.g. in a vaccination context, or for generation of multispecific binding complexes. In an icosahedral complex 1-60 different peptides or polypeptides may be displayed, e.g. 2, 3, 4, 5 or more. In an octahedral complex 1-24 different peptides or polypeptides may be displayed, e.g. 2, 3, 4, 5 or more.

Display of up to 60 or up to 24 different peptides or polypeptides on a single molecule allows for creation of designer multifunctional molecules. For example, new multifunctional protein or multienzyme systems can be assembled. Simultaneous display of several different epitopes is possible, for example B-cell, T-helper and CTL epitopes, important in vaccine design. Targeting sequences or proteins such as antibodies or receptors may be incorporated. Some proteins carrying unique/specific chemical or post-translational modifications, such as fluorophores, glycosylations, phosphorylations, etc, may be incorporated. Complex assemblies carrying some or all of the above are possible.

Analogous embodiments are provided where, instead of displaying a peptide or polypeptide by means of fusion with an E2 core protein portion, the peptide is displayed by means of non-covalent interaction with the E2 core protein portion. This may be via the peripheral subunit-binding domain. For instance, the peptide or polypeptide to be displayed may be provided as a conjugate or fusion with an E3 component or a portion thereof which interacts non-covalently with the peripheral subunit-binding domain of the E2 protein which assembles into the core. This could also include use of the E3-binding protein found in the E2 cores of eukaryotic PDH complexes, which is responsible for tight non-covalent binding of E3 in those complexes.

One or more components of the complex or core may be modified to reduce or abolish enzymatic activity. This may be especially desirable where the complex displaying one or more peptides or polypeptides is intended for administration to an individual, e.g. a patient in need of therapeutic treatment or vaccination. The enzymatic activity of the E2 protein of pyruvate dehydrogenase (PDH) complex of *Bacillus stearothermophilus* (CoA acyltransferase activity) may be ablated by mutation of residues His398 and Thr346. Corresponding mutations can be made in the E2 core proteins of other 2-oxo acid dehydrogenase multienzyme complexes employed in accordance with the invention (see e.g. de Kok et al. (1998) Biochimica et Biophysica Acta 1385: 353-366 for review, also Griffin and Chuang (1990) Journal of Biological Chemistry 265: 13174-13180; Hendle et al., (1995) Biochemistry 34: 4287-4298, Russell and Guest (1990) Biochemical Journal 269: 443-450 and Russell and Guest (1991) Proceedings of the Royal Society of London Series B 243: 155-160).

Any one multiprotein complex may display 60 copies (icosahedral) or 24 copies (octahedral) of a peptide or polypeptide, and each multiprotein complex in the population or library may display a different peptide or polypeptide. In a population or library of complexes, the complexes collectively display a range of different peptides or polypeptides, providing diversity in sequence. Diversity in sequence provides for diversity in one or more properties, e.g. binding ability, allowing for processes of selection and screening to be performed to identify and obtain one or more peptides or polypeptides with a desired property.

An E2 core or complex displaying a peptide or polypeptide in accordance with the present invention may be additionally modified (e.g. by introduction of a cysteine residue) so as to permit chemical attachment of another molecule to the scaffold. This may be useful, for example, in targeting the complex to cells or cell compartments by means of a displayed binding molecule. In another embodiment, it could be used to conceal molecules within the cavity of the E2 core to promote their uptake by target cells when the complex is endocytosed.

A complex may be immobilised on a support by means of one or more peptides or polypeptides displayed on its surface, or by appropriate chemical modification. One or more other peptides or polypeptides on the complex may then bind an additional molecule. This could be valuable in using the complex to display ligands, e.g. in ELISA assays.

Peptides tend to be short, and may be about 40 amino acids in length or less, preferably about 35 amino acids in length or less, more preferably about 30 amino acids in length, or less, more preferably about 25 amino acids or less, more preferably about 20 amino acids or less, more preferably about 15 amino acids or less, more preferably about 10 amino acids or less, or 9, 8, 7, 6, 5 or less in length. Peptides of the invention may be about 10-20 amino acids, about 10-30 amino acids, about 20-30 amino acids or about 30-40 amino acids in length. To include a single epitope, a preferred length of peptide may be 5-6 amino acids.

Polypeptides are larger. A polypeptide may include a full length protein or a protein domain, a folded unit of function e.g. capable of binding to a ligand.

A peptide or polypeptide fused to an E2 component as disclosed is identified as "heterologous" by not being present in the native component at the site of fusion.

Peptides employed in the present invention may be generated as fragments of a polypeptide, for instance a protein of interest for which epitope mapping is desired, or may be provided (especially in the context of a population or library for screening) as random or semi-random sequences. Nucleic acid encoding random or semi-random amino acid sequences may be provided within a sequence encoding a fusion with a component of a multiprotein complex in accordance with standard techniques.

Polypeptides for display may include antibody molecules (e.g. scFv fragments), enzymes, protein domains such as receptor domains, enzyme inhibitors, protein ligands, targeting proteins and so on.

A method of producing a truncated E2 protein or fusion protein as disclosed, may comprise providing nucleic acid encoding the relevant protein in a suitable expression system (e.g. host cell) and causing or allowing production of the encoded product by expression from the encoding nucleic acid. Where the nucleic acid is within a host cell, the method may comprise culturing the host cell under suitable conditions.

Following production of the encoded product it may be recovered from the expression system, e.g. host cell or host cell culture, and isolated and/or purified.

Complexes may be assembled as disclosed.

Purification of the fusion components from the host cells can be achieved using standard purification techniques, for example including size exclusion chromatography, affinity chromatography, ion exchange chromatography, sucrose gradient ultracentrifugation. Assembly of E2 components into icosahedral/octahedral complexes occurs upon expression and folding of the fusions *in vivo*. Assembly can be performed *in vitro,* for instance of E2 scaffolds displaying two or more different fusion peptides/polypeptides. It can also be performed after suitable chemical modification. For example, E2 rendered fluorescent by modification with fluorescein isothiothyarate (FITC) or any other fluorophor can be refolded with an E2/peptide fusion. *In vitro* assembly involves unfolding or disassembly of mixtures of isolated E2 proteins (e.g. fusions) and refolding of the mixture in appropriate conditions. The *B*. *stearothermophilus* PDH E2 component can be unfolded in the presence of 6M guanidine hydrochloride and refolded by dialysis into aqueous buffer pH7.0 (Lessard et al., (1998) European Journal of Biochemistry, 258: 491-501). Molecular chaperones may be included, especially for other E2 components (Wynn et al., (1994) Biochemistry 33: 8962-8968). *In vivo,* assembly of E2 scaffolds displaying more than one fusion may require coexpression of the desired E2 fusions within the same host cell by making the desired nucleic acid vectors. This may be performed by already published methods such as expression of the different fusion proteins from the same vector (Scrutton et al., (1990) Proceedings of the Royal Society of London Series B 242: 217-224), or from different vectors with different origins of replication (Deonarain et al., (1992) Biochemistry 31: 1498-1504).

A population or library of complexes displaying different peptides or polypeptides may be provided by expression from host cells plated out to allow for screening to identify clones producing a peptide or polypeptide of interest, e.g. with a desired property that may be identified by screening.

Screening by plating out may be performed using standard protocols for screening cDNA libraries, e.g. as described in Current Protocols in Molecular Biology (Ausubel et al. eds., John Wiley & Sons (1994); Skalka and Shapiro (1976) Gene 1: 65-79; Kemp and Cowman (1981) Proceedings of the National Academy of Sciences (USA) 78: 4520-4524; Helfman et al., (1983) Proceedings of the National Academy of Sciences (USA) 80: 31-35).

Desired properties for which peptides or polypeptides may be screened include: ability to bind a specific binding member (ligand) of interest (e.g. antibody molecule or receptor), enzymatic activity, and so on.

Another aspect of the present invention provides a method of obtaining one or more peptides or polypeptides with a desired property, the method including screening a library of peptides or polypeptides displayed as disclosed for one or more peptides or polypeptides which have the desired property, and selecting one or more peptides or polypeptides of the library which have the desired property.

Another aspect of the present invention provides a method of obtaining one or more peptides or polypeptides containing an epitope bound by a specific binding member (ligand), the method including bringing into contact a library of peptides or polypeptides displayed as disclosed and a specific binding member (ligand), and selecting one or more peptides or polypeptides of the library able to bind said specific binding member (ligand).

One utility of such a method is to obtain a peptide containing an epitope that is immunologically cross-reactive with an antigen of interest (useful for example in vaccination contexts).

Another aspect of the present invention therefore provides a method of obtaining one or more peptides containing an epitope immunologically cross-reactive with an epitope in an antigen of interest, the method including bringing into contact a library of peptides as disclosed and an antibody molecule able to bind said antigen of interest, and selecting one or more peptides of the library able to bind said antibody molecule.

Such methods may include bringing into contact a library of peptides and a plurality of antibody molecules or other specific binding members collectively able to bind a plurality of targets of interest, e.g. strains of a virus or other pathogen. In one embodiment, said plurality of antibody molecules is derived from sera of individuals infected with the pathogen.

As noted, a library of the invention comprises peptides or polypeptides displayed on multiprotein complexes. Following screening and identification of complexes of interest, nucleic acid may be taken from clones expressing the complexes of interest. Nucleic acid with the sequence of nucleic acid taken from a clone expressing a complex displaying a said selected peptide may be used in production of such a peptide by means of expression (using recombinant DNA technology as standard in the art and discussed further below).

A peptide with the amino acid sequence of a said selected peptide may be provided in isolated form, e.g. after its production by expression from encoding nucleic acid. Another possibility is for one or more peptides in accordance with the present invention to be provided by peptide synthesis, using any of a variety of techniques at the disposal of the ordinary person skilled in the art. Peptides may be synthesized using standard peptide chemistry such as by the common method employing Fmoc ((9-fluoromethyloxycarbonyl) t-BU (*tert*-butyl)), as described in Atherton and Sheppard (1989), Solid Phase Peptide Synthesis, a Practical Approach, IRL Press, Oxford.

A plurality of peptides each with the amino acid sequence of a different selected peptide may be provided in isolated form, individually or in a mixture.

A convenient way of producing a peptide or polypeptide according to the present invention is to express nucleic acid encoding it, by use of the nucleic acid in an expression system. This applies to peptides and polypeptides obtained by a method of screening as discussed, and also the components of the relevant multiprotein complex themselves, including the fusion protein comprising the heterologous peptide or polypeptide.

A method of making a peptide or polypeptide (as disclosed) may include expression from nucleic acid encoding the peptide or polypeptide (generally in the case of a multiprotein complex component the polypeptide is fused to a heterologous peptide or polypeptide sequence). This may conveniently be achieved by growing a host cell in culture, containing such a vector, under appropriate conditions which cause or allow expression of the polypeptide. Peptides and polypeptides may also be expressed in *in vitro* systems, such as reticulocyte lysate.

Nucleic acid is generally provided as DNA or RNA, though may include one or more nucleotide analogues, and may be wholly or partially synthetic. Nucleic acid molecules and vectors may be provided in isolated and/or purified form, e.g. in substantially pure or homogeneous form. The term isolated may be used to reflect all these possibilities. Where a DNA sequence is specified, e.g. with reference to a figure, unless context requires otherwise the RNA equivalent, with U substituted for T where it occurs, is encompassed.

Where it is desired to express a peptide or polypeptide from encoding nucleic acid, the nucleic acid includes appropriate regulatory control sequences. Suitable vectors can be chosen or constructed, containing appropriate regulatory sequences, including promoter sequences, terminator fragments, polyadenylation sequences, enhancer sequences, marker genes and other sequences as appropriate. Vectors may be plasmids, viral e.g. 'phage, or phagemid, as appropriate. For further details see, for example, Molecular Cloning: a Laboratory Manual: 2nd edition, Sambrook et al., (1989) Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Ausubel et al. eds., John Wiley & Sons (1994).

Systems for cloning and expression of a polypeptide in a variety of different host cells are well known. Suitable host cells include bacteria, eukaryotic cells such as mammalian and yeast, and baculovirus systems. Mammalian cell lines available in the art for expression of a heterologous polypeptide include Chinese hamster ovary cells, HeLa cells, baby hamster kidney cells, COS cells and many others. A common, preferred bacterial host is *E*. *coli*.

A host cell may contain nucleic acid as disclosed herein. The nucleic acid of the invention may be integrated into the genome (e.g. chromosome) of the host cell. Integration may be promoted by inclusion of sequences which promote recombination with the genome, in accordance with standard techniques. The nucleic acid may be on an extra-chromosomal vector within the cell.

A method which may include introducing the nucleic acid into a host cell. The introduction, which may (particularly for *in vitro* introduction) be generally referred to without limitation as "transformation", may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. For bacterial cells, suitable techniques may include calcium chloride transformation, electroporation and transfection using bacteriophage. As an alternative, direct injection of the nucleic acid could be employed. Marker genes such as antibiotic resistance or sensitivity genes may be used in identifying clones containing nucleic acid of interest, as is well known in the art.

The introduction may be followed by causing or allowing expression from the nucleic acid, e.g. by culturing host cells (which may include cells actually transformed although more likely the cells will be descendants of the transformed cells) under conditions for expression of the gene, so that the encoded peptide or polypeptide is produced. If the peptide or polypeptide is expressed coupled to an appropriate signal leader peptide it may be secreted from the cell into the culture medium. Following production by expression, a peptide or polypeptide may be isolated and/or purified from the host cell and/or culture medium, as the case may be, and subsequently used as desired, e.g. in the formulation of a composition which may include one or more additional components, such as a pharmaceutical composition which includes one or more pharmaceutically acceptable excipients, vehicles or carriers (e.g. see below).

A peptide or polypeptide may be used as an immunogen or otherwise in obtaining binding antibodies. Antibodies are useful in purification and other manipulation of polypeptides and peptides, diagnostic screening and therapeutic contexts, including passive immunisation.

One or more antibody molecules containing a binding site able to bind a peptide or polypeptide (optionally displayed as a fusion with a component of a multiprotein complex) may be obtained by a method including bringing into contact a population of antibody molecules and a said peptide or polypeptide, and selecting one or more antibody molecules of the population able to bind said peptide or polypeptide.

The method may involve bringing the population of antibodies into contact with a plurality of peptides or polypeptides.

As noted, the peptides or polypeptides may be provided in a fusion with additional amino acids.

The peptide(s) or polypeptide(s) (optionally displayed as a fusion with a component of a multiprotein complex as disclosed) may be administered to a non-human mammal to bring them into contact with a population of antibody molecules produced by the mammal's immune system, then one or more antibody molecules able to bind the peptide(s) or polypeptide(s) may be taken from the mammal, or cells producing such antibody molecules may be taken from the mamma 1.

The mammal may be sacrificed.

If cells are taken from the mammal, antibody.molecules may be taken from said cells or descendants thereof. Such descendants in particular may include hybridoma cells.

Antibodies useful in screening in accordance with the invention may be isolated, in the sense of being free from contaminants such as antibodies able to bind other polypeptides and/or free of serum components. Monoclonal antibodies are preferred for some purposes, though polyclonal antibodies may be used. Indeed, polyclonal mixtures able to bind one or more peptides or polypeptides are preferred for some purposes. A mixture of different antibodies may be able to bind one or more peptides or polypeptides. Such a mixture may be provided in a composition including at least one additional component, such as a pharmaceutically acceptable excipient or vehicle.

Antibodies to one or more peptides or polypeptides may be obtained or raised. A method of evoking an immune response (B and/or T cell) may include administering a peptide or polypeptide or mixture of peptides or polypeptides (wherein the peptide or polypeptide may be displayed as a fusion with a component of a multiprotein complex as disclosed, or may have been obtained by screening a library as disclosed) to a mammal in order to raise an antibody response. In a therapeutic or prophylactic context the mammal may be human or non-human. For the production of antibodies or antibody-producing cells to be isolated and used for any of a variety of purposes, a step of sacrificing a non-human mammal may be included. Such a non-human mammal may be for example mouse, rat, rabbit, dog, cat, pig, horse, donkey, goat, sheep, camel, Old World monkey, chimpanzee or other primate. Antibodies may be obtained from immunised animals using any of a variety of techniques known in the art, and screened, preferably using binding of antibody to peptide or polypeptide of interest. For instance, Western blotting techniques or immunoprecipitation may be used (Armitage et al., (1992) Nature, 357:80-82).

The production of polyclonal and monoclonal antibodies is well established in the art. Monoclonal antibodies can be subjected to the techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP-A-184187, GB-A-2188638 or EP-A-239400. Humanised antibodies in which CDRs from a non-human source are grafted onto human framework regions, typically with the alteration of some of the framework amino acid residues, may provide antibodies which are less immunogenic than the parent non-human antibodies. A hybridoma producing a monoclonal antibody may be subject to genetic mutation or other changes , which may or may not alter the binding specificity of antibodies produced. Cloning and expression of chimeric antibodies are described in EP-A-0120694 and EP-A-0125023.

Antibodies useful in aspects and embodiments of the invention as disclosed may be modified in a number of ways. Indeed the term "antibody molecule" should be construed as covering any binding substance having a binding domain of an antibody. Example antibody fragments, capable of binding an antigen or other binding partner are the Fab fragment consisting of the VL, VH, Cl and CH1 domains; the Fd fragment consisting of the VH and CH1 domains; the Fv fragment consisting of the VL and VH domains of a single arm of an antibody; the dAb fragment which consists of a VH domain; isolated CDR regions and F(ab')2 fragments, a bivalent fragment including two Fab fragments linked by a disulphide bridge at the hinge region. Single chain Fv fragments are also included.

In screening for binding between peptide or polypeptide and a specific binding member such as an antibody molecule, the reactivities of antibodies on peptide/polypeptide may be determined by any appropriate means. Tagging with individual reporter molecules is one possibility. The reporter molecules may directly or indirectly generate detectable, and preferably measurable, signals. The linkage of reporter molecules may be directly or indirectly, covalently, e.g. via a peptide bond or non-covalently. Linkage via a peptide bond may be as a result of recombinant expression of a gene fusion encoding antibody and reporter molecule.

One favoured mode is by covalent linkage of each antibody with an individual fluorochrome, phosphor or laser dye with spectrally isolated absorption or emission characteristics. Suitable fluorochromes include fluorescein, rhodamine, phycoerythrin and Texas Red. Suitable chromogenic dyes include diaminobenzidine.

Other reporters include macromolecular colloidal particles or particulate material such as latex beads that are coloured, magnetic or paramagnetic, and biologically or chemically active agents that can directly or indirectly cause detectable signals to be visually observed, electronically detected or otherwise recorded. These molecules may be enzymes which catalyse reactions that develop or change colours or cause changes in electrical properties, for example. They may be molecularly excitable, such that electronic transitions between energy states result in characteristic spectral absorptions or emissions. They may include chemical entities used in conjunction with biosensors. Biotin/avidin or biotin/streptavidin and alkaline phosphatase detection systems may be employed.

The mode of determining binding is not a feature of the present invention and those skilled in the art are able to : choose a suitable mode according to their preference and general knowledge.

As noted already, peptides, polypeptides, multiprotein complexes displaying peptides or polypeptides, and antibodies, also nucleic acid encoding a peptide or polypeptide may be formulated into compositions, and are useful in pharmaceutical contexts. These compositions may include, in addition to one of the above substances, a pharmaceutically acceptable excipient, carrier, buffer, stabiliser or other materials well known to those skilled in the art. Such materials should be non-toxic and should not interfere with the efficacy of the active ingredient. The precise nature of the carrier or other material may depend on the route of administration, e.g. oral, intravenous, cutaneous or subcutaneous, nasal, intramuscular, intraperitoneal routes.

Compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

An adjuvant may be included, such as alum, oil-in-water emulsions or Freund's Adjuvant (Complete or Incomplete). Cytokines may be used to potentiate immunogenicity of the peptide or polypeptide composition. An adjuvant, cytokine or other immunogenicity boosting accompaniment may not be required. The pyruvate dehydrogenase (PDH) complex of *Bacillus stearothermophilus,* for example, is highly immunogenic.

Further aspects and embodiments of the present invention will be apparent to those skilled in the art in view of the present disclosure, including the following experimental exemplification with reference to the accompanying figures.

### Brief Description of the Figures

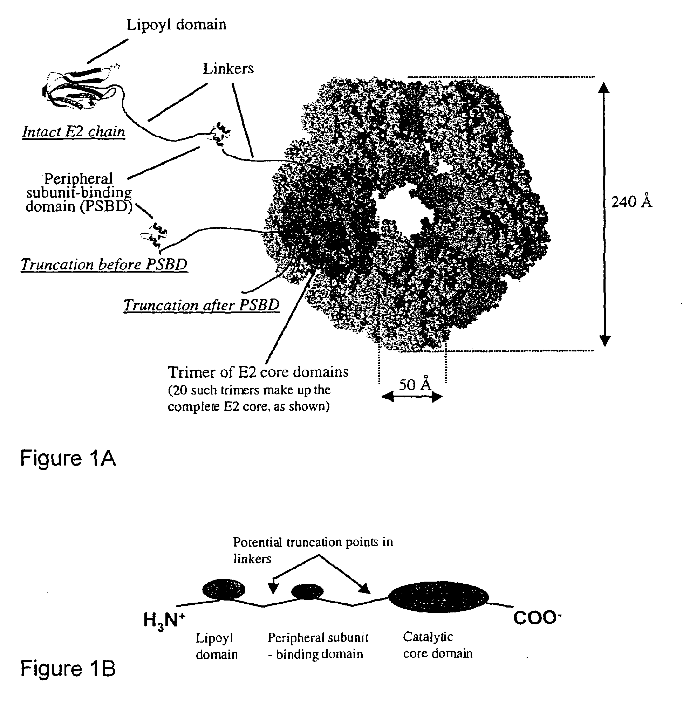

Figure 1A gives a schematic representation of the icosahedral *B*. *stearothermophilus* E2 core. Only 3 of the 60 E2 chains are shown with their N-terminal segments in place for clarity (Izard et al., (1999) Proceedings of the National Academy of Sciences USA 96: 1240-1245; Dardel et al., (1993) Journal of Molecular Biology 229: 1037-1048; Kalia et al., (1993) Journal of Molecular Biology 230: 323-341).
Figure 1B gives a schematic representation of the *stearothermophilus* E2 chain indicating some potential truncation points for insertion of heterologous peptide/polypeptide to display on the E2 core assembly.
Figure 2 shows a schematic representation of the vector pETHE2DISP used in the experiments described below for peptide and polypeptide display.
Figure 3 shows a schematic representation of the vector pETE2DISP used in the experiments described for peptide and polypeptide display.

### EXPERIMENTAL

### Vectors for the Expression of Foreign Peptides/Polypeptides on the E2 Core Surface

Two *E. coli* expression vectors were designed and constructed for the expression of foreign peptides and polypeptides at the N-terminus of the E2 core domain of pyruvate dehydrogenase (PDH) complex of *Bacillus stearothermophilus* and thus on the surface of the E2 core:
1. pETHE2DISP. This vector allows over-expression of foreign peptide/polypeptide between a histidine tag and the E2 core. The histidine tag and a thrombin cleavage site lie on the N-terminal side of the fusion protein, to allow subsequent excision of the tag if desired. Three unique restrictions sites allow directional ligation of the insert DNA.
2. pETE2DISP. Same as above, but without the histidine tag and the thrombin cleavage site. The foreign protein is expressed directly at the N-terminus of the E2 core domain.

### Expression and Display on the Surface of E2

As examples, two peptides were displayed on the surface of the E2 core: MAL1 with the sequence (NANP)₃ (SEQ ID NO. 11) and MAL2 with the sequence NDDSYIPSAEKI (SEQ ID NO. 12). These two peptides were previously used to exemplify the display of peptides on the surface of filamentous bacteriophage virions (Greenwood et al., (1991) Journal of Molecular Biology 220: 821-827; Willis et al., (1993) Gene 128: 79-83).

Using the plasmid pETHE2DISP, constructs were made to generate E2MAL1 and E2MAL2. *E. coli* cells expressing E2DISP, E2MAL1 and E2MAL2 were lysed and the cell lysates run on 12% SDS PAGE. The gel was electroblotted onto nitrocellulose transfer membranes and immunoblotted using antibodies raised against bacteriophage fd and antibodies raised against a hybrid fd/fdMAL1 bacteriophage (Greenwood et al., (1991) Journal of Molecular Biology 220: 821-827), respectively. The immunoblots were developed with goat anti-rabbit IgG-alkaline phosphatase conjugate and BCIP/NBT substrate. Only E2MAL1 was recognised by the antibodies raised against fd/fdMAL1, demonstrating that the added epitope was functional.

### EXAMPLE 1

### Construction Of E2 Expression Vectors For Display.

The E2 core domain of the *Bacillus stearothermophilus* PDH complex is the protein domain responsible for forming the 60-meric icosahedral scaffold. Two vectors were constructed to facilitate fusion of foreign polypeptides to its N-terminus. The two vectors are referred to as pETHE2DISP, and pETE2DISP. The two vectors are derived from the Novagen *E. coli* expression vectors pet15b and pet11d as well as the *Bacillus stearothermophilus* E2p expression vector pETBstE2 described in Lessard et al., (1998) European Journal of Biochemistry, 258: 491-501.

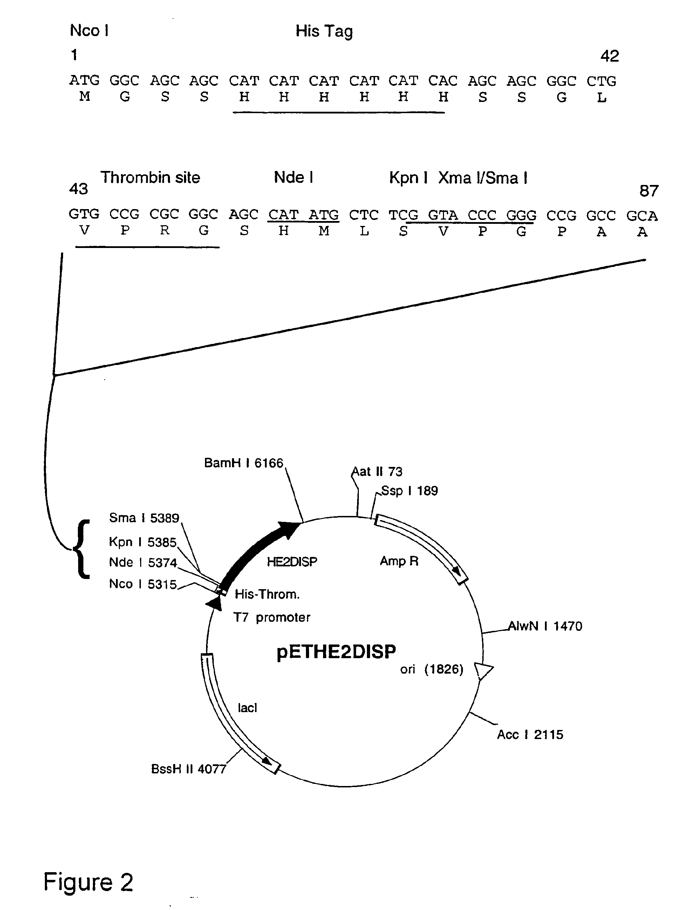

The portion of DNA encoding the E2 core domain and a portion of the linker sequence were PCR amplified from pETBstE2 using the two oligonucleotides with SEQ ID NO. 1 and SEQ ID NO. 2. SEQ ID NO. 1 introduces four unique restriction sites at the 5' end of the E2 core domain. The amplified PCR product was digested with the restriction enzymes Nde1 and BamH1 as was the commercial Novagen vector pet15b The digested pet15b vector was treated with calf intestinal alkaline phosphatase (CIAP) from Boehringer Mannheim. Both DNA products were purified from gel electrophoresis and ligated with T4 DNA ligase (Pharmacia LKB Biotechnology Inc.). The ligation mixture was introduced into *E*. *coli* XL1-Blue cells by electroporation and colonies hosting circular plasmid DNA selected for on ampicillin LB media plates. Plasmid DNA from selected colonies was purified using the appropriate Qiagen kits and sequenced using the appropriate oligonucleotide primers, from the vector T7 promoter up to the BamH1 restriction site. The plasmid with the desired sequence is defined as pETHE2DISP (Figure 2).

The plasmid pETHE2DISP has the following properties: five unique sites for introduction of foreign DNA at the 5' end of the E2 core gene (Ncol, Nde1, Sma1, Xma1, and Kpn1); the foreign DNA can be inserted such that the foreign polypeptide is expressed between a histidine tag followed by a specific thrombin digest site and the E2 core gene; the foreign DNA can be inserted such that the foreign DNA is expressed directly at the N-terminus (without the Histidine tag): and the vector allows direct heterologous expression of the recombinant gene in the appropriate *E*. *coli* cells.

The plasmid pETE2DISP was derived from pETHE2DISP. Plasmid pETHE2DISP was digested with Nco1 and Xma1. Oligonucleotide sequences SEQ ID NO. 3 and SEQ ID NO. 4 were annealed and ligated into the digested pETHE2DISP with a ten fold molar excess of oligonucleotide insert to plasmid in the ligation mixture. The desired circular plasmid was selected for as described above. The plasmid pETE2DISP has the same properties as pETHE2DISP except that the Nde1 restriction site has been removed and the optional histidine tag-thrombin digest site is no longer available as part of the vector.

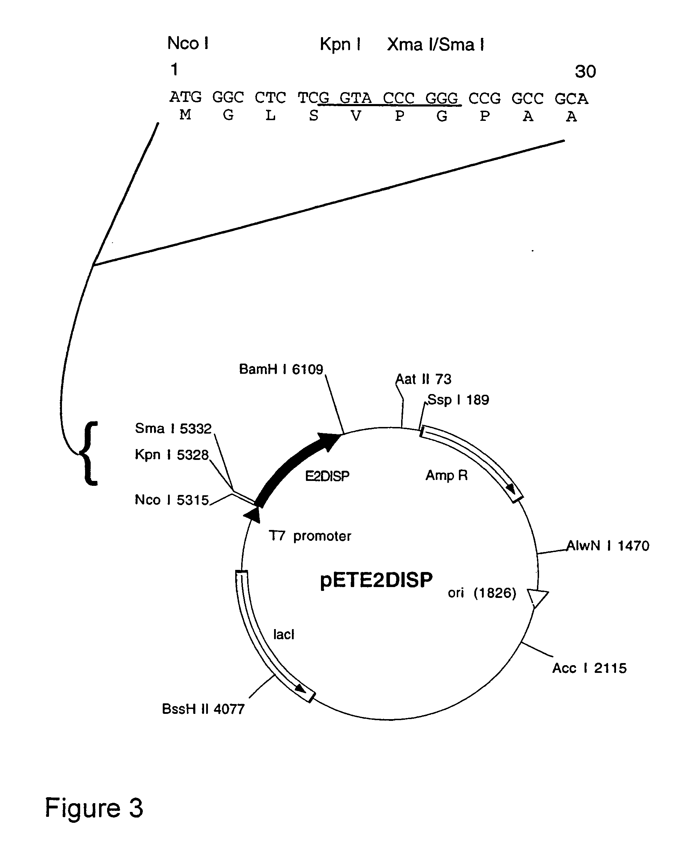

A schematic representation of the vector pETE2DISP is shown in Figure 3.

### EXAMPLE 2

### Construction of pETE2MAL1 & pETE2MAL2

The plasmids were derived from pETHE2DISP as described for pETE2DISP. Oligonucleotide sequences SEQ ID NO. 5 and SEQ ID NO. 6 were used to generate pETMAL1 and SEQ ID NO. 7 and SEQ ID NO. 8 were used to generate pETMAL2. All constructs were confirmed by DNA sequencing with appropriate oligonucleotide primers.

These vectors were used to display the peptides MAL1 and MAL2 as noted above, and described in more detail below.

### EXAMPLE 3

### Construction of pETE2EGFP and display of functional GFP

A sequence encoding for enhanced green flourescent protein (EGFP) was PCR amplified from the commercial vector pEGFP-1 (from Clontech) using oligonucleotides SEQ ID NO. 9 and SEQ ID NO. 10. Both the PCR product and the plasmid pETHE2DISP were digested with Nco1 and Xma1. The digested vector was treated with CIAP. Ligation and selection for circular plasmid was performed as described in Example 1. The correct sequence for pETE2EGFP was confirmed by DNA sequencing.

Cells expressing EGFP-E2 fusion were shown to fluoresce in contrast to cells just expressing E2. These results provide indication that the EGFP protein is folding correctly on the E2 scaffold.

Cell lysate from cells expressing the EGFP-E2 fusion were submitted to SDS-PAGE (12.5% acrylamide) and blotted on to PVDF membrane as described for peptides in Example 5. Specific binding of polyclonal E2DISP antisera (described in Example 6) and monoclonal anti-GFP antibodies (supplied by Clontech) were tested using alkaline phosphatase detection as described in Example 5.

Blots developed with E2DISP antisera and anti-GFP antibodies detected the same single protein band corresponding to a protein with a molecular weight just under 60 kDa. In contrast, cell lysates of cells expressing E2DISP, E2MAL1, and E2MAL2 ran on the same SDS-PAGE gels but separate lanes were only detected on the blots developed with E2DISP antisera at positions corresponding to proteins with molecular weights of just under 30 kDa. As the predicted molecular weight of the EGFP-E2 protein is 56.4 kDa, these results show that the active GFP detected by fluorescence is covalently attached to the E2, and that there is no observable degradation of the fusion protein.

### EXAMPLE 4

### Expression & purification of E2 core domain fusion proteins

### 4.1 Expression of the E2 core domain fusion proteins

The plasmids pETHE2DISP, pETE2DISP, pETE2MAL1, pETE2MAL2 and pETE2EGFP were used to express the E2 core domain fusion proteins: HE2DISP, E2DISP, E2MAL1, E2MAL2 and E2EGFP, respectively. Expression of the E2 core domain fusion proteins was achieved as described in Lessard et al., (1998) European Journal of Biochemistry, 258: 491-501.

Briefly, a sample (5 ml) of an overnight culture (100 ml) of *E. coli* BL21(DE3)pLysS cells freshly transformed with desired vector was used to inoculate 1 litre of LB medium. When the A₆₀₀ reached 0.7, the inducer IPTG was added to a final concentration of 1 mM. After a further 4 hours, the cells were harvested by centrifugation. All cells were grown at 37°C except for those expressing E2EGFP, these were grown at 29°C.

### 4.2 Purification of the E2 core domain fusion proteins

E2DISP, E2MAL1 and E2MAL2 were purified as described in Lessard et al., (1998) European Journal of Biochemistry, 258: 491-501. All steps were performed at 4°C unless otherwise stated. Protein concentration was estimated by the Coomassie dye-binding method, except for pure proteins which were estimated by amino acid analysis. Column fractions were analysed by means of SDS-PAGE (12.5% acrylamide) using the Pharmacia PhastSystemJ. Freshly grown *E. coli* cells (derived from 5 litres of culture) were resuspended (2 ml/g of wet cells) in 20 mM Tris-HCl, pH 8.5, containing 10 mM EDTA, 1 mM PMSF and 1 mM 1,10-phenanthroline (buffer A), and disrupted at 131 x 10⁶ Pa in a French press (SLM-AMINCO). Cell debris were removed by centrifugation at 95000 *g* for 20 min, and the supernatant was subjected to ammonium sulphate fractionation. The protein that was precipitated between 35-65% saturation was redissolved in buffer A and dialysed against two changes of 2 litres of the same buffer. The supernatant was applied (at a flow rate of 1 ml/min) in three separate batches onto a Pharmacia HiLoad^{™} 16/10 Q Sepharose^{™} High Performance anion exchange column previously equilibrated with 20 mM Tris-HCl, pH 8.5, containing 1 mM EDTA and 0.02% sodium azide (buffer B). The E2 fusion protein was eluted with a linear gradient of 0-0.6 M NaCl in buffer B at a flow rate of 1 ml/min over 120 ml. Fractions (5 ml) containing the E2 fusion protein were pooled, concentrated to approximately 20 mg/ml using a Centriprep30 (Amicon Ltd), and loaded (at a flow rate of 0.5 ml/min) in batches of 1.2 ml onto a Pharmacia Sepharose^{™} CL-2B gel filtration column (1.6 x 70 cm) previously equilibrated with 100 mM potassium phosphate, pH 7.0, containing 0.02% sodium azide (buffer C). Fractions (1 ml) containing the pure E2 fusion protein were pooled. The pooled fractions of E2 fusion protein were concentrated to approximately 4 mg/ml, flash frozen in liquid nitrogen and stored at -80°C. The identity of the protein was confirmed by N-terminal sequencing and mass spectrometry.

HE2DISP was purified by affinity chromatography in its denatured form. *E. coli* cells expressing HE2DISP were resuspended in 6MGuHCl 0.1M potassium phosphate pH8.0 (buffer D) at 5ml/gram cells. After one hour of mixing the cell lysate was pelleted by centrifugation for 20 minutes at 10000xg. The supernatant was then loaded on to a 4ml column of Ni-NTA (nickel-nitrilo-tri-acetic acid) resin (from Qiagen) at a flow rate of 15ml/hr. The column was then washed with ten column volumes of buffer D, followed by a ten column volume wash with 6MGuHCl 0.1M potassium phosphate pH6.3. The HE2DISP was eluted by washing the column with 15ml 6MGuHCl 0.1M potassium phosphate pH5.9 and 15ml of 6MGuHCl 0.1M potassium phosphate pH4.5. Fractions containing HE2DISP as determined by SDS-PAGE were pooled.

### EXAMPLE 5

### Epitope display on the E2 core

E2DISP, E2 MAL1 and E2MAL2 were run on an SDS-PAGE (12.5 % acrylamide) using the Pharmacia PhastSystem^{™}, and blotted on to PVDF membrane using standard Pharmacia protocols. Specific binding of antibodies to the electroblotted proteins was tested using alkaline phosphatase detection. For this protocol the following solutions were used: TBS, 20 mM Tris, 500mM NaCl, adjusted to pH 7.5 with HCl; blocking buffer, 50g/l Marvel^{™} dried skimmed milk in TBS; primary antibody solution, primary antibody e.g. anti-bacteriophage fd or anti-fd-Mal1 in a solution of blocking buffer; secondary antibody solution, goat anti-rabbit IgG-alkaline phosphatase conjugate diluted 1:2000 times in blocking buffer; and development buffer, one tablet of SIGMA-FASTJ BCIP/NBT alkaline phosphatase substrate (5-bromo-4-chloro-3-indolyl phosphate/nitro blue tetrazolium tablets) in 10ml deionized water. The blot was submitted to 2 x 10min washes in blocking buffer followed by a 1 hr incubation in blocking buffer. The blot was then incubated overnight in primary antibody solution, followed by 4 x 10min washes in blocking buffer and 1 x 10 min wash in TBS. The blot was then incubated with secondary antibody solution for 1hr and consequently washed with blocking buffer 4 x 10min. The blot was then washed in TBS for ten minutes, and incubated with the development buffer until bands begin to appear. Development was stopped with 2 x 5 min deioinised water washes.

### EXAMPLE 6

### Generation of immune response in vivo raising antibodies to peptides displayed on the E2 scaffold

Purified E2DISP and E2MAL1 were injected into separate New Zealand white rabbits in the presence of Freund's complete adjuvant for the primary immunisation and with Freund's incomplete adjuvant for all subsequent boosts. The rabbits received boosts at days 14, 28, 42, 56 and 70 after the primary immunisation. Test bleeds were taken on days 35, 49, 63 and 77. Antisera from all test bleeds from rabbits injected with either E2DISP or E2MAL1 were capable of specific recognition of the *B*. *stearothermophilus* E2 constructs in *E*. *coli* cell lysates generated as described in Example 4.1. Preimmunisation sera from the same rabbits showed no antibody binding. Specific binding of antibodies from the antisera was detected using the protocols described in Example 5. A 1 in 50,000 dilution of the E2DISP or E2MAL1 antisera as the primary antibody source was sufficient to detect antibodies specific to the E2 constructs from antisera taken on day 49.

Antisera from rabbits immunised with E2MAL1 was subjected to an affinity purification step. Purified E2DISP was immobilised on cyanogen bromide - activated Sepharose 4B beads (Sigma) using the manufacturers recommended protocols. 1ml of E2MAL1 antisera was then incubated for one hour at 4°C with 0.5ml of the E2DISP-coupled Sepharose beads. The beads were then precipitated, removed and replaced with a fresh 0.5 ml of E2DISP-coupled Sepharose beads. This was repeated for four changes of E2DISP-coupled Sepharose beads. A 1 in 50,000 dilution of the affinity-purified E2MAL1 antisera as the primary antibody source was sufficient to detect antibodies specific to the E2MAL1 construct but not to the E2DISP, E2MAL2, and E2EGFP constructs, suggesting that antibodies specific to the E2MAL1 fusion had been raised in the rabbits.

### EXAMPLE 7

### Multiple display of different polypeptides on the E2 surface

Chimeric E2 icosahedral cores, with up to three different molecules displayed on the same 60-mer core were generated by *in vitro* refolding of cell lysates from cells expressing EGFP-E2, E2MAL1, and HE2DISP.

*E.coli* cells BL21pLysS, transformed separately with pETHE2DISP, pETE2MAL1, and pETE2EGFP and grown at 28°C in LB media were induced with IPTG when they reached an absorbance (600nm) of 0.8. The cells were harvested three hours after induction and denatured by incubation and mixing for two hours in 6 M guanidine hydrochloride, 1mM DL-dithiothreitol, 50mM potassium phosphate pH 8.0. The cell lysates were then pelleted by centrifugation for 20 minutes at 1000xg. The following mixtures of denatured cell-lysate supernatant were then prepared: 1) E2EGFP + E2MAL1; 2) HE2DISP + E2MAL1; 3) HE2DISP + E2MAL1+ E2EGFP, and 4) E2EGFP. 330 ml, 50 ml and 30 ml cell culture volume equivalents of E.coli cells transformed with E2EGFP, E2MAL1 and HE2DISP respectively, were used to prepare the above mixtures. These mixtures were then "renatured" by dialysis into 1mM DL-dithiothreitol, 50mM potassium phosphate pH 8.0. Dialysis was performed with four changes of 2 litres dialysis buffer (50mM potassium phosphate pH 8.0) over 24 hours at 4°C for every 20ml of cell lysate mixture. The renatured cell lysates were then centrifuged for 20m minutes at 1000xg and the supernatant retained.

The renatured cell lysate from 4 was then added to the renatured cell lysate 2 to give the following three renatured cell lysate mixtures:
1') [E2EGFP-E2MAL1];
2') [HE2DISP-E2MAL1] + E2EGFP;
3') [HE2DISP-E2MAL1-E2EGFP].

1ml of Ni-NTA agarose resin (Pharmacia) was then incubated with the three mixtures 1', 2' and 3' for one hour with immidazole and NaCl added to 20mM and 0.3M concentrations, respectively. From this a 0.5 ml column was poured for each mixture. The columns were washed consecutively with 5ml, 3ml, and 3ml of 20 mM, 50 mM and 250 mM imidazole in 0.3M NaCl, 1mMDTT, 50mM Kphos pH 8.0, respectively. The samples eluted from the Ni-NTA columns with 250 mM imidazole were tested for binding to MAL1-specific antibodies (anti-fd-Mal1 as described in Example 5) and E2DISP polyclonal antibodies (described in Example 6).

E2 protein constructs were only detected for samples 2' and 3' which contained the histidine-tagged E2DISP protein, HE2DISP. Whereas in both samples 2' and 3' the MAL1 epitope of the E2MAL1 construct was detected, only in sample 3' was the high molecular weight E2EGFP construct detected.

These results provide indication that chimeric E2 scaffolds presenting both the histidine tag and the MAL1 epitope were generated from mixture 2 and E2 scaffolds presenting all three polypeptides, the histidine tag, the MAL1 epitope and the EGFP polypeptide were generated from mixture 3.

### SEQUENCES

SEQ ID NO. 1:
   GGGAATTCCATATGCTCTCGGTACCCGGGCCGGCCGCAGCGGAG
SEQ ID NO. 2:
   GCGGGATATCCGGATATAGT
SEQ ID NO. 3:
   CATGGGCCTCTCGGTAC
SEQ ID NO. 4:
   CCGGGTACCGAGAGGCC
SEQ ID NO. 5:
   CATGGGCAATGCAAACCCGAACGCAAACCCGAATGCAAACCCGC
SEQ ID NO. 6:
   CCGGGCGGGTTTGCATTCGGGTTTGCGTTCGGGTTTGCATTGCC
SEQ ID NO. 7:
   CATGGGCAACGATGATAGCTATATCCCGAGCGCGGAAAAAATCC
SEQ ID NO. 8:
   CCGGGGATTTTTTCCGCGCTCGGGATATAGCTATCATCGTTGCC
SEQ ID NO. 9:
   CGGTCGCCACCATGGTGAGCAAG
SEQ ID NO. 10:
   GGTGGATCCCGGGTCCCGCGGTACCGT
SEQ ID NO. 11:
   NANPNANPNANP
SEQ ID NO. 12:
   NDDSYIPSAEKI

## Claims

1. A population or library of assembled cores of a 2-oxo acid dehydrogenase multienzyme complex comprising a fusion protein comprising:
(i) a portion of a 2-oxo acid dehydrogenase multienzyme complex E2 protein component which assembles into a core structure of a 2-oxo acid dehydrogenase multienzyme complex,
wherein said portion comprises the catalytic core subunit of the native E2 component, and
(ii) a heterologous peptide or heterologous polypeptide,
the population or library collectively displaying a plurality of different peptides or polypeptides.

2. A population or library according to claim 1 wherein an assembled core in said population or library displays more than one different peptide or polypeptide.

3. A population or library according to claim 1 or 2 wherein the fusion protein comprises a linker between the portion of the E2 protein component and the heterologous peptide or heterologous polypeptide.

4. A population or library according to any one of claims 1 to 3 wherein the heterologous peptide or polypeptide is at the N-terminus of the portion of the E2 protein component or, if present, said linker.

5. A population or library according to claim 3 or claim 4 wherein the linker comprises the native E2 linker region present in native E2 protein between the peripheral subunit-binding domain and the catalytic core domain.

6. A method of obtaining one or more peptides or polypeptides with a desired property, the method comprising
screening a population or library according to any one of claims 1 to 5 for one or more peptides or polypeptides which have the desired property, and
selecting one or more peptides or polypeptides which have the desired property.

7. A method of obtaining one or more peptides or polypeptides containing an epitope to which a ligand binds, the method comprising
bringing into contact a population or library of assembled cores displaying peptides or polypeptides according to any one of claims 1 to 5 and a ligand, and
selecting one or more peptides or polypeptides that bind said ligand.

8. A method according to claim 7 for obtaining one or more peptides containing an epitope immunologically cross-reactive with an epitope in an antigen of interest, the method comprising
bringing into contact a population or library of assembled cores displaying peptides and an antibody molecule able to bind said antigen of interest, and
selecting one or more peptides that bind said antibody molecule.

9. A population or library of assembled cores of a 2-oxo acid dehydrogenase multienzyme complex, each said core comprising
(i) a portion of a 2-oxo acid dehydrogenase multienzyme complex E2 protein component including the peripheral sub-unit binding domain which assembles into a core structure of a 2-oxo acid dehydrogenase multienzyme complex and which includes the peripheral sub-unit binding domain, and
(ii) a heterologous peptide or polypeptide in a conjugate or fusion with an E3 component of 2-oxo acid dehydrogenase which interacts non-covalently with the peripheral subunit-binding domain of the E2 protein which assembles into the core,
the population or library collectively displaying a plurality of different peptides or polypeptides.

10. A population or library according to claim 9 wherein heterologous peptide or heterologous polypeptide is fused to the E3 component.

11. A population or library according to claim 9 or claim 10 further comprising the E3 binding protein.

12. A population or library according to any one of claims 1 to 5 or 9 to 11 wherein one or more of said portions of the E2 component are modified by mutation to reduce or abolish enzymatic activity of said E2 component,
wherein said one or more portions are modified by mutation of residues corresponding to His398 and Thr346 of the E2 component of the PDH complex of B. stearothermophilus.

13. A population or library according to any one of claims 1 to 5 or 9 to 11 wherein a core or complex of said population or library is modified by the introduction of a cysteine residue to permit chemical attachment of another molecule to the assembled core.

14. A population or library according to claim 13 wherein one or more additional molecules are attached to the assembled core.

15. A population or library according to any one of claims 1 to 5 or 9 to 14 wherein an assembled core of said population or library is immobilised on a support.

16. A population or library according to claim 15 wherein the core is immobilised by means of one or more peptides or polypeptides displayed on its surface or by chemical modification.

17. A population or library according to any one of claims 1 to 5 or 9 to 16 comprising an assembled core of a 2-oxo acid dehydrogenase multienzyme complex, said assembled core comprising,
a first fusion protein comprising
(i) a portion of a 2-oxo acid dehydrogenase multienzyme complex E2 protein component which assembles into a core structure of a 2-oxo acid dehydrogenase multienzyme complex, and
(ii) a first heterologous peptide or polypeptide, the core further comprising a second fusion protein comprising a second heterologous peptide or polypeptide,
wherein the first heterologous peptide or polypeptide is different from the second heterologous peptide or polypeptide.

18. Use of an assembled core comprising one or more fusion proteins comprising
(i) a portion of a 2-oxo acid dehydrogenase multienzyme complex E2 protein component which assembles into a core structure of a 2-oxo acid dehydrogenase multienzyme complex, and
(ii) a heterologous peptide or polypeptide,
to produce a population or library according to any one of claims 1 to 5 or 9 to 16.

## Patentansprüche

1. Population oder Reihe von zusammengesetzten Kernen eines 2-Oxo-Säure-Dehydrogenase-Multienzym-Komplexes mit einem Schmelzprotein, mit:
(i) einem Teil einer 2-Oxo-Säure-Dehydrogenase-Multienzymkomplex-E2-Protein-Komponente, die sich aufbaut in eine Kernstruktur eines 2-Oxo-Säure-Dehydrogenase-Multienzym-Komplexes, wobei dieser Teil die katalytische Kern-Untereinheit der natürlichen E2-Komponente aufweist, und
(ii) einem heterogenen Peptid oder heterogenen Polypeptid,
wobei die Population oder Reihe kollektiv eine Vielzahl von unterschiedlichen Peptiden oder Polypeptiden wiedergibt.

2. Population oder Reihe nach Anspruch 1, bei der ein zusammengesetzter Kern in dieser Population oder Reihe mehr als ein unterschiedliches Peptid oder Polypeptid wiedergibt.

3. Population oder Reihe nach Anspruch 1 oder 2, bei der das Schmelzprotein einen Linker zwischen dem Teil der E2-Proteinkomponente und dem heterogenen Peptid oder heterogenen Polypeptid aufweist.

4. Population oder Reihe nach einem der Ansprüche 1 bis 3, bei der das heterogene Peptid oder Polypeptid ein N-Terminus des Teils der E2-Protein-Komponente oder, falls vorhanden, dieses Linkers ist.

5. Population oder Reihe nach Anspruch 3 oder Anspruch 4, bei der der Linker die natürliche E2-Linker-Region aufweist, die in dem natürlichen E2-Protein zwischen dem peripheren Untereinheit-Bindebereich und dem katalytischen Kernbereich vorhanden ist.

6. Verfahren zum Gewinnen eines oder mehrerer Peptide oder Polypeptide mit einer erwünschten Eigenschaft, wobei das Verfahren umfasst
Sichtung einer Population oder Reihe nach jedem der Ansprüche 1 bis 5 auf ein oder mehrere Peptide oder Polypeptide, welche die erwünschte Eigenschaft haben, und
Auswahl eines oder mehrerer Peptide oder Polypeptide, welche die gewünschte Eigenschaft haben.

7. Verfahren zum Gewinnen eines oder mehrerer Peptide oder Polypeptide, die ein Epitop enthalten, an welches sich ein Ligand bindet, wobei das Verfahren umfasst
das Inkontaktbringen einer Population oder Reihe von zusammengesetzten Kernen, die Peptide oder Polypeptide nach jedem der Ansprüche 1 bis 5 und einen Ligand darstellen, und
Auswahl eines oder mehrerer Peptide oder Polypeptide, die den Ligand binden.

8. Verfahren nach Anspruch 7 zum Erzielen eines oder mehrerer Peptide, die ein Epitop enthalten, das immonologisch mit einem Epitop in einem Antigen von Interesse querreaktiv ist, wobei das Verfahren umfasst
das Inkontaktbringen einer Population oder Reihe von zusammengesetzten Kernen, die Peptide und ein Antikörpermolekül darstellen, das in der Lage ist, dieses Antigen von Interesse zu binden, und
Auswahl eines oder mehrerer Peptide, welche dieses Antikörpermolekül binden.

9. Population oder Reihe von zusammengesetzten Kernen eines 2-Oxo-Säure-Dehydrogenase-Mulienzym-Komplexes, wobei jeder Kern aufweist:
(i) einen Teil einer 2-Oxo-Säure-Dehydrogenase-Mulienzym-Komplex-E2-Proteinkomponente, einschließlich des peripheren Untereinheit-Bindebereiches, der sich in eine Kernstruktur eines 2-Oxo-Säure-Dehydrogenase-Mulienzym-Komplexes zusammensetzt und den peripheren Untereinheit-Bindungsbereich umfasst, und
(ii) ein heterogenes Peptid oder Polypeptid in einer Kongugation oder einer Fusion mit einer E3-Komponente der 2-Oxo-Säure-Dehydrogenase, die nicht-kovalent mit dem peripheren Untereinheit-Bindebereich des E2-Proteins, das sich in den Kern zusammensetzt, interagiert, wobei die Population oder Reihe kollektiv eine Vielzahl von unterschiedlichen Peptiden oder Polypeptiden wiedergibt.

10. Population oder Reihe nach Anspruch 9, bei der das heterogene Peptid oder heterogene Polypeptid zur E3-Komponente geschmolzen wird.

11. Population oder Reihe nach Anspruch 9 oder 10, die ferner das E3-Bindeprotein aufweist.

12. Population oder Reihe nach jedem der Ansprüche 1 bis 5 oder 9 bis 11, bei der ein oder mehrere dieser Anteile der E2-Komponente durch Mutation modifiziert wird bzw. werden, um eine enzymatische Aktivität dieser E2-Komponente zu reduzieren oder aufzuheben, wobei der eine oder mehrere Anteile durch Mutation von Resten entsprechend His 398 und Thr 346 der E2 Komponente des PDH Komplexes von B. Stearothermophil modifiziert werden.

13. Population oder Reihe nach jedem der Ansprüche 1 bis 5 oder 9 bis 11, wobei ein Kern oder Komplex dieser Population oder Reihe durch Einführung eines Zysteinrestes modifiziert wird, um eine chemische Anbindung eines weiteren Moleküls an den zusammengesetzten Kern zuzulassen.

14. Population oder Reihe nach Anspruch 13, bei der ein Molekül oder mehrere zusätzliche Moleküle am zusammengesetzten Kern angegliedert ist bzw. sind.

15. Population oder Reihe nach jedem der Ansprüche 1 bis 5 oder 9 bis 14, bei der ein zusammengesetzter Kern der Population oder Reihe auf einem Träger unbeweglich gemacht ist.

16. Population oder Reihe nach Anspruch 15, bei der der Kern mittels eines oder mehrerer Peptide oder Polypeptide unbeweglich gemacht ist, das bzw. die auf dessen Oberfläche ausgebreitet ist bzw. sind, oder durch chemische Modifikation.

17. Population oder Reihe nach jedem der Ansprüche 1 bis 5 oder 9 bis 16 mit einem zusammengesetzten Kern eines 2-Oxo-Säure-Dehydrogenase-Multienzym-Komplexes, wobei der Kern ein erstes Schmelzprotein umfasst, bestehend aus
(i) einem Teil einer 2-Oxo-Säure-Dehydrogenase-Multienzym-Komplex E2 Proteinkomponente, welche sich zu einer Kernstruktur eines 2-Oxo-Säure-Dehydrogenase-Multienzym-Komplexes zusammenfügt, und
(ii) einem ersten heterologen Peptid oder Polypeptid, wobei der Kern ferner ein zweites Schmelzprotein hat, das ein zweites heterologes Peptid oder Polypeptid aufweist,
wobei das erste heterologene Peptid oder Polypeptid sich von dem zweiten heterologen Peptid oder Polypeptid unterscheidet.

18. Verwendung eines zusammengesetzten Kerns mit einem oder mehreren Schmelzproteinen, umfassend
(i) einen Teil einer 2-Oxo-Säure-Dehydrogenase-Multienzym-Komplex-E2-Protein-Komponente, welche sich in eine Kernstruktur eines 2-Oxo-Säure-Dehydrogenase-Multienzym-Komplexes aufbaut, und
(ii) ein heterologes Peptid oder Polypeptid, um eine Population oder Reihe nach jedem der Ansprüche 1 bis 5 oder 9 bis 16 zu erzeugen.

## Revendications

1. Population ou bibliothèque de noyaux assemblés d'un complexe multienzymatique de 2-oxo acide déshydrogénase comprenant une protéine de fusion comprenant :
(i) une partie d'un composant protéique E2 du complexe multienzymatique de 2-oxo acide déshydrogénase qui s'assemble dans une structure de noyau d'un complexe multienzymatique de 2-oxo acide déshydrogénase,
dans laquelle ladite partie comprend la sous-unité de noyau catalytique du composant E2 natif, et
(ii) un peptide hétérologue ou un polypeptide hétérologue,
la population ou la bibliothèque présentant collectivement une pluralité de peptides ou de polypeptides différents.

2. Population ou bibliothèque selon la revendication 1, dans laquelle un noyau assemblé dans ladite population ou bibliothèque présente plus d'un peptide ou polypeptide différent.

3. Population ou bibliothèque selon la revendication 1 ou 2, dans laquelle la protéine de fusion comprend un lieur entre la partie du composant protéique E2 et le peptide hétérologue ou le polypeptide hétérologue.

4. Population ou bibliothèque selon l'une quelconque des revendications 1 à 3, dans laquelle le peptide ou le polypeptide hétérologue se trouve à l'extrémité N-terminale de la partie du composant protéique E2 ou, s'il est présent, dudit lieur.

5. Population ou bibliothèque selon la revendication 3 ou la revendication 4, dans laquelle le lieur comprend la région de lieur du composant E2 natif présente dans la protéine E2 native entre le domaine de liaison de sous-unité périphérique et le domaine de noyau catalytique.

6. Procédé d'obtention d'un ou plusieurs peptides ou polypeptides avec une propriété souhaitée, le procédé comprenant
le criblage d'une population ou d'une bibliothèque selon l'une quelconque des revendications 1 à 5 pour un ou plusieurs peptides ou polypeptides qui ont la propriété souhaitée, et
la sélection d'un ou plusieurs peptides ou polypeptides qui ont la propriété souhaitée.

7. Procédé d'obtention d'un ou plusieurs peptides ou polypeptides contenant un épitope auquel se lie un ligand, le procédé comprenant
la mise en contact d'une population ou d'une bibliothèque de noyaux assemblés présentant des peptides ou des polypeptides selon l'une quelconque des revendications 1 à 5 et d'un ligand, et
la sélection d'un ou plusieurs peptides ou polypeptides qui lient ledit ligand.

8. Procédé selon la revendication 7 destiné à l'obtention d'un ou plusieurs peptides contenant un épitope qui présente une réaction croisée immunologique avec un épitope dans un antigène d'intérêt, le procédé comprenant
la mise en contact d'une population ou d'une bibliothèque de noyaux assemblés présentant des peptides et d'une molécule d'anticorps capable de lier ledit antigène d'intérêt, et
la sélection d'un ou plusieurs peptides qui lient ladite molécule d'anticorps.

9. Population ou bibliothèque de noyaux assemblés d'un complexe multienzymatique de 2-oxo acide déshydrogénase, chaque dit noyau comprenant
(i) une partie d'un composant protéique E2 du complexe multienzymatique de 2-oxo acide déshydrogénase incluant le domaine de liaison de sous-unité périphérique qui s'assemble dans une structure de noyau d'un complexe multienzymatique de 2-oxo acide déshydrogénase et qui inclut le domaine de liaison de sous-unité périphérique, et
(ii) un peptide ou un polypeptide hétérologue dans un conjugué ou une fusion avec un composant E3 de 2-oxo acide déshydrogénase qui interagit de façon non covalente avec le domaine de liaison de sous-unité périphérique de la protéine E2 qui s'assemble dans le noyau,
la population ou la bibliothèque présentant collectivement une pluralité de peptides ou de polypeptides différents.

10. Population ou bibliothèque selon la revendication 9, dans laquelle un peptide hétérologue ou un polypeptide hétérologue est fusionné au composant E3.

11. Population ou bibliothèque selon la revendication 9 ou la revendication 10, comprenant en outre la protéine liante E3.

12. Population ou bibliothèque selon l'une quelconque des revendications 1 à 5 ou 9 à 11, dans laquelle une ou plusieurs desdites parties du composant E2 sont modifiées par mutation afin de réduire ou de supprimer l'activité enzymatique dudit composant E2,
dans laquelle ladite/lesdites une ou plusieurs parties sont modifiées par mutation de résidus correspondant à His398 et à Thr346 du composant E2 du complexe PDH de B. *stearothermophilus.*

13. Population ou bibliothèque selon l'une quelconque des revendications 1 à 5 ou 9 à 11, dans laquelle un noyau ou complexe de ladite population ou bibliothèque est modifié par l'introduction d'un résidu de cystéine pour permettre une fixation chimique d'une autre molécule sur le noyau assemblé.

14. Population ou bibliothèque selon la revendication 13, dans laquelle une ou plusieurs molécules supplémentaires sont fixées sur le noyau assemblé.

15. Population ou bibliothèque selon l'une quelconque des revendications 1 à 5 ou 9 à 14, dans laquelle un noyau assemblé de ladite population ou bibliothèque est immobilisé sur un support.

16. Population ou bibliothèque selon la revendication 15, dans laquelle le noyau est immobilisé au moyen d'un ou plusieurs peptides ou polypeptides présentés sur sa surface ou par une modification chimique.

17. Population ou bibliothèque selon l'une quelconque des revendications 1 à 5 ou 9 à 16 comprenant un noyau assemblé d'un complexe multienzymatique de 2-oxo acide déshydrogénase, ledit noyau assemblé comprenant,
une première protéine de fusion comprenant
(i) une partie d'un composant protéique E2 du complexe multienzymatique de 2-oxo acide déshydrogénase qui s'assemble dans une structure de noyau d'un complexe multienzymatique de 2-oxo acide déshydrogénase, et
(ii) un premier peptide ou polypeptide hétérologue, le noyau comprenant en outre une seconde protéine de fusion comprenant un second peptide ou polypeptide hétérologue,
dans laquelle le premier peptide ou polypeptide hétérologue est différent du second peptide ou polypeptide hétérologue.

18. Utilisation d'un noyau assemblé comprenant une ou plusieurs protéines de fusion comprenant
(i) une partie d'un composant protéique E2 du complexe multienzymatique de 2-oxo acide déshydrogénase qui s'assemble dans une structure de noyau d'un complexe multienzymatique de 2-oxoacide déshydrogénase, et
(ii) un peptide ou un polypeptide hétérologue,
afin de produire une population ou une bibliothèque selon l'une quelconque des revendications 1 à 5 ou 9 à 16.
